# EUROPEAN PATENT SPECIFICATION

(11) **EP 4 199 868 B1**
(45) Date of publication and mention of the grant of the patent: **06.08.2025**
(21) Application number: 21765778.2
(22) Date of filing: 11.08.2021
(51) Int. Cl.: A61F 5/02

(54) **SACROILIAC ORTHOSIS**
SAKROILIAKALORTHESE
ORTHÈSE SACRO-ILIAQUE

(30) Priority: 19.08.2020 US 202063067764 P
(43) Date of publication of application: 28.06.2023
(73) Proprietor: DJO, LLC, Carlsbad, CA 92010 (US)
(72) Inventor: MADDEN, Dave, Carlsbad, CA 92010 (US); NATHANSON, Jeremy, Vista, CA 92084 (US)
(74) Representative: Abel & Imray LLP
(86) International application number: PCT/US2021/045487
(87) International publication number: WO 2022/039987

(56) References cited:
- WO-A1-2009/054354
- US-A1- 2016 324 678
- US-A1- 2019 374 366
- US-A1- 2020 237 547

## Description

### BACKGROUND

The present disclosure relates to sacroiliac orthosis, methods of using the same to provide pelvic sacral support, and/or methods of manufacturing such orthoses.

A sacroiliac belt can be worn around the upper hips of an individual to exert a compressive force around the pelvic girdle and to stabilize and reduce strain in the sacroiliac joint. There are a number of commercially available orthoses intended to stabilize and immobilize the lumbar and sacral region of the body for use in the treatment of lower back pain. Such devices are often employed for treating pain associated with conditions such as lumbar sprain, spinal stenosis, disc herniation, as well as pain and discomfort associated with degenerative spinal disorders. Nevertheless, many of these devices do not fully address spinal concerns.

Due to the variability of individuals in terms of size, weight and anatomy, ensuring optimal fit and comfort can be challenging. There remains a need for sacroiliac belts that can be easily adjusted to ensure proper fit, customizable support, and requisite immobilization to the necessary regions of the back when the device is worn.

One type of sacroiliac belt disclosed in the art includes an elastic inner part that wraps around the user's hips and a non-elastic outer part that encircles the inner part to establish an overall non-elastic sacroiliac belt. U.S. Patent No. 4,572,167 discloses a sacroiliac belt, with elastic panels and a non-elastic cinch strap encircling the panels. Another sacroiliac belt of this type includes elastic Neoprene to wrap around the user's hips and non-elastic Nylon to wrap around the Neoprene. However, this sacroiliac belt is not recommended for wearing directly against the skin because the closed-cell structure of Neoprene causes heat build-up which can lead to skin irritation.

Another type of prior sacroiliac belt is generally elastic, which allows good compression of the sacroiliac joint. However, while such elastic sacroiliac belts can provide a certain measure of lumbar support, such elastic sacroiliac belts will not typically provide sufficient compression to specifically limit motion of the sacroiliac joint below its very low, normal range of motion, e.g., approximately 3 degrees. In this way, an overall elastic sacroiliac belt can give a false sense of security to the user because the actions that open the sacroiliac joint, i.e., bending, lifting, and twisting, can force the joint open beyond its normal range of motion even with such elastic sacroiliac belts in place. In other words, elastic sacroiliac belts do not create a stop point at the end of the normal range of motion of the sacroiliac joint. As ligaments are essentially non-elastic, movement of the sacroiliac joint beyond its normal range will stress the ligaments and potentially re-injure the joint.

U.S. Patent Publication No. 2019/0374366A1 describes a sacroiliac belt with internal distractions. The design includes a belt having an inner belt configured to encircle a waist portion of a user. The inner belt has a first end, a second end, and a length that extends from the first end to the second end. Notably, the belt also includes a plurality of pockets coupled to the internal side of the inner belt, configured to receive distraction devices.

U.S. Patent No. 9,402,758 describes a supporter constructed from a single sheet of knitted fabric.

International PCT Patent Application No. WO-A-2009/054354 describes a length-adjustable belt in which, to both ends of a band-shaped member (a) made of a stretchable material or a material having been subjected to a stretching treatment, either a band-shaped member (B) made of a non-stretchable material or a material having been subjected to a non-stretching treatment or a band-shaped member (B) made of a stretchable material having a lower stretching ratio than the above-described band-shaped member (a) or a material having been subjected to the same stretching treatment is formed and which is wound around and tightens up the body side at the position almost below the anterior superior iliac spine of the iliac bone constituting the pelvis, wherein a pressing member is provided on the back face of the above-described band-shaped member (a) at the site being nearly in contact with the area between the posterior superior iliac spine and the posterior inferior iliac spine when the belt is wound around the body, and locking members are provided at both ends of the belt.

U.S. Patent Publication No. US-A-2020/237547 describes orthopedic braces and associated methods for treatment of lower back injuries and chronic back pain. An orthopedic brace may include a pair of back panels, a pair of front panels, and a closure system. A lateral end of each front panel is releasably coupleable to a lateral end of each back panel at a desired angle. A ventral end of each front panel includes an attachment provision configured to allow one of the front panels to releasably attach to the other of the front panels generally over an abdomen of a wearer. The brace also includes a pocket on the ventral end of at least one of the front panels, the pocket configured to temporarily receive a hand of the wearer to aid in donning the orthopedic brace.

U.S. Patent Publication No. US-A-2016/324678 describes an orthopedic device in the form of a lumbar support which includes first and second elongate belt members, an anatomically shaped plate, and a closure system connecting the belt members to the plate. The closure system is arranged to move the belt members relative to the plate, and connects to the belt members via a flexible belt attachment which removably secures to the belt members. The closure system includes tensioning elements corresponding to the belt members, and a pulley system connecting to the tensioning elements. The closure system is slidably mounted to the plate and arranged to the belt members relative to the plate between opposed linear directions. The plate has various contours which provide pressure distribution over a lumbar region of a back. Anatomically shaped and resiliently formed handles secure to the tensioning elements and the belt members.

Accordingly, a need exists for improved sacroiliac belts that provide sufficient support and stabilization to one or more sacroiliac joints of a wearer. Additionally, there remains a need for adjustable belts to enhance the fit of such belts on the wearer.

### SUMMARY

In some embodiments, an orthopedic sacroiliac brace is provided. The brace includes a belt portion. The brace includes a first posterior sacral panel coupled to the belt portion and configured to be disposed directly posterior of a first posterior side of an ilium of a user. The brace includes a second posterior sacral panel coupled to the belt portion and configured to be disposed directly posterior of a second posterior side of the ilium. The brace includes an anterior abdominal panel coupled to the belt portion and configured to be disposed against an anterior portion of an abdomen of the user, the anterior abdominal panel characterised by: a central portion having a substantially circular or ellipsoid form factor and configured to be disposed directly anterior of at least the sacrum of the user; and first and second extensions that each extend away from the central portion in opposite directions and are configured to be disposed directly anterior of a respective sacroiliac joint and at least a portion of the corresponding side of the ilium. Adjustably tightening the belt portion around the user causes the first and second posterior sacral panels to apply respective anterior forces to the first and second posterior sides of the ilium, and the anterior abdominal panel to simultaneously apply a posterior force to the abdomen of the user, thereby providing adjustable stabilizing support, compression and/or alignment to a sacroiliac joint of the user.

### BRIEF DESCRIPTION OF THE DRAWINGS

A more complete appreciation of the subject matter of the present disclosure and of the various advantages thereof can be realized by reference to the following detailed description in which reference is made to the accompanying drawings in which:
**FIG. 1** illustrates an anatomy of a hip, pelvis and lower back of a human body, in accordance with some embodiments;
**FIG. 2** illustrates a wireframe diagram showing anterior and posterior directions of force and/or support provided by one or more portions of a sacroiliac orthosis, in accordance with some embodiments;
**FIG. 3** illustrates the wireframe diagram of **FIG.** 2 with additional circumferential directions of force and/or support provided by one or more portions of a sacroiliac orthosis, in accordance with some embodiments;
**FIG. 4A** illustrates an outer view of a sacroiliac orthosis, in accordance with some embodiments;
**FIG. 4B** illustrates a semi-transparent outer view of the sacroiliac orthosis of **FIG. 4A** providing partial visibility of one or more inner features of the sacroiliac orthosis;
**FIG. 5A** illustrates an inner view of the sacroiliac orthosis of **FIGs. 4A** and **4B****,** in accordance with some embodiments;
**FIG. 5B** illustrates a semi-transparent inner view of the sacroiliac orthosis of **FIG. 5A** providing partial visibility of one or more outer features of the sacroiliac orthosis;
**FIG. 6A** illustrates an outer view of a sacroiliac orthosis having a removable anterior abdominal panel, in accordance with some embodiments;
**FIG. 6B** illustrates a semi-transparent outer view of the sacroiliac orthosis of **FIG. 6A** providing partial visibility of one or more inner features of the sacroiliac orthosis;
**FIG. 7A** illustrates an outer view of a sacroiliac orthosis with supportive plates removed therefrom, in accordance with some embodiments;
**FIG. 7B** illustrates a semi-transparent outer view of the sacroiliac orthosis of **FIG. 7A** providing partial visibility of one or more inner features of the sacroiliac orthosis;
**FIG. 8** illustrates a perspective wireframe view of a sacroiliac orthosis having ends of its belt portion coupled to one another, in accordance with some embodiments;
**FIG. 9** illustrates a position of supportive panels within the wireframe view of the sacroiliac orthosis of **FIG. 8****,** in accordance with some embodiments;
**FIG. 10** is a wireframe illustration of a substantially anterior view of a sacroiliac orthosis, in accordance with some example embodiments;
**FIG. 11** is a wireframe illustration of a substantially posterior view of the sacroiliac orthosis of **FIG. 10****,** in accordance with some example embodiments;
**FIG. 12** is a flowchart related to a method of using a sacroiliac orthosis; and
**FIG. 13** is a flowchart related to a method of manufacturing a sacroiliac orthosis.

### DETAILED DESCRIPTION

The present disclosure relates to sacroiliac orthoses, methods of using such orthoses to provide pelvic sacral support, and associated methods of manufacturing such orthoses.

The invention is defined in the claims. Any subject-matter that is disclosed herein, but which is not defined in the claims, does not form part of the invention.

### General Interpretive Principles

Various aspects of the novel systems, apparatuses, and methods are described more fully hereinafter with reference to the accompanying drawings. The teachings disclosure may, however, be embodied in many different forms and should not be construed as limited to any specific structure or function presented throughout this disclosure. Rather, these aspects are provided so that this disclosure will be thorough and complete, and will fully convey the scope of the disclosure to those skilled in the art. Based on the teachings herein one skilled in the art should appreciate that the scope of the disclosure is intended to cover any aspect of the novel systems, apparatuses, and methods disclosed herein, whether implemented independently of or combined with any other aspect of the disclosure. For example, a system or an apparatus may be implemented, or a method may be practiced using any one or more of the aspects set forth herein. In addition, the scope of the disclosure is intended to cover such a system, apparatus or method which is practiced using other structure, functionality, or structure and functionality in addition to or other than the various aspects of the disclosure set forth herein. It should be understood that any aspect disclosed herein may be set forth in one or more elements of a claim. Although some benefits and advantages of the preferred aspects are mentioned, the scope of the disclosure is not intended to be limited to particular benefits, uses, or objectives. The detailed description and drawings are merely illustrative of the disclosure rather than limiting, the scope of the disclosure being defined by the appended claims and equivalents thereof.

With respect to the use of plural vs. singular terms herein, those having skill in the art can translate from the plural to the singular and/or from the singular to the plural as is appropriate to the context and/or application. The various singular/plural permutations may be expressly set forth herein for sake of clarity.

When describing an absolute value of a characteristic or property of a thing or act described herein, the terms "substantial," "substantially," "essentially," "approximately," and/or other terms or phrases of degree may be used without the specific recitation of a numerical range. When applied to a characteristic or property of a thing or act described herein, these terms refer to a range of the characteristic or property that is consistent with providing a desired function associated with that characteristic or property.

In those cases where a single numerical value is given for a characteristic or property, it is intended to be interpreted as at least covering deviations of that value within one significant digit of the numerical value given.

If a numerical value or range of numerical values is provided to define a characteristic or property of a thing or act described herein, whether or not the value or range is qualified with a term of degree, a specific method of measuring the characteristic or property may be defined herein as well. In the event no specific method of measuring the characteristic or property is defined herein, and there are different generally accepted methods of measurement for the characteristic or property, then the measurement method should be interpreted as the method of measurement that would most likely be adopted by one of ordinary skill in the art given the description and context of the characteristic or property. In the further event there is more than one method of measurement that is equally likely to be adopted by one of ordinary skill in the art to measure the characteristic or property, the value or range of values should be interpreted as being met regardless of which method of measurement is chosen.

It will be understood by those within the art that terms used herein, and especially in the appended claims (e.g., bodies of the appended claims) are intended as "open" terms unless specifically indicated otherwise (e.g., the term "including" should be interpreted as "including but not limited to," the term "having" should be interpreted as "having at least," the term "includes" should be interpreted as "includes but is not limited to," etc.).

Various modifications to the implementations described in this disclosure can be readily apparent to those skilled in the art, and generic principles defined herein can be applied to other implementations without departing from the scope of this disclosure. Thus, the disclosure is not intended to be limited to the implementations shown herein but is to be accorded the widest scope consistent with the claims, the principles and the novel features disclosed herein. The word "exemplary" is used exclusively herein to mean "serving as an example, instance, or illustration." Any implementation described herein as "exemplary" is not necessarily to be construed as preferred or advantageous over other implementations.

### Several Example Embodiments

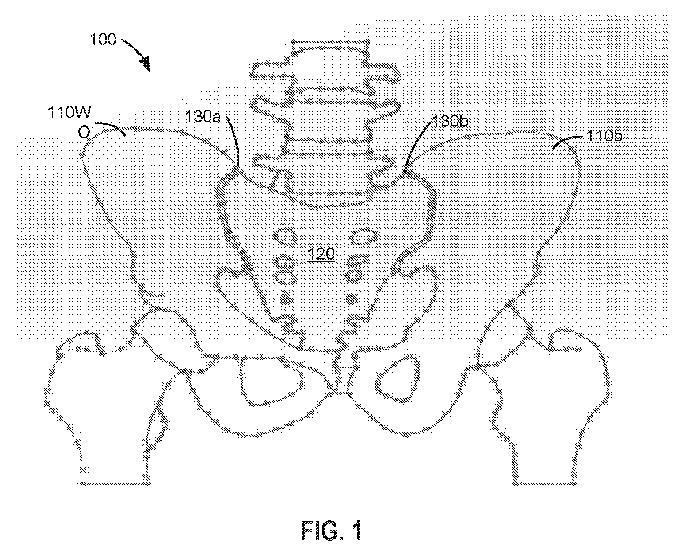

**FIG. 1** illustrates an anatomy of a hip, pelvis and lower back of a human body, in accordance with some embodiments. For example, a hip 100 is illustrated, comprising a sacrum 120 (forming a bottom of the backbone) and an ilium having a first posterior side 110a disposed immediately adjacent a first side of sacrum 120 and a second posterior side 110b disposed immediately adjacent a second side of sacrum 120 opposite the first side of sacrum 120. A first sacroiliac (SI) joint 130a is formed between first posterior side 110a of ilium and the immediately adjacent first side of sacrum 120 and a second SI joint 130b is formed between second posterior side 110b of the ilium and the immediately adjacent second side of sacrum 120.

Actions that can open first and/or second SI joint(s) 130a, 130b include bending, lifting, and/or twisting motions of the low back. However, the normal range of motion of first and second SI joints 130a, 130b (e.g., a range of motion of first and/or second sides 110a, 110b of ilium with respect to the corresponding immediately adjacent first and/or second sides of sacrum 120) is very low, e.g., approximately 3°. Accordingly, where such actions force first and/or second SI joint 130a, 130b open beyond their normal range(s) of motion, stress or damage to, and/or injury or reinjury of, the ligaments holding the first and/or second SI joints 130a, 130b together can occur. Accordingly, a need exists for a sacral orthosis, brace and/or belt configured to provide support and/or stabilization specifically to first and/or second SI joints 130a, 130b.

Accordingly, this disclosure contemplates sacroiliac orthoses, braces or belts having one or more removable and/or movable panels disposed such that they are configured to create pressure in the coronal plane of the hip/pelvis region as circumferential pressure is applied to the orthosis, brace and/or belt. This pressure compresses the tissues of the hip and, thereby, stabilizes and maintains proper alignment of one or both of SI joints 130a, 130b in that coronal plane. Inventive embodiments described herein benefit, in part, from a surprising discovery that sacroiliac orthoses, braces and/or belts, as described herein, provide a unique level of support and/or stabilization specifically to first and/or second SI joints 130a, 130b.

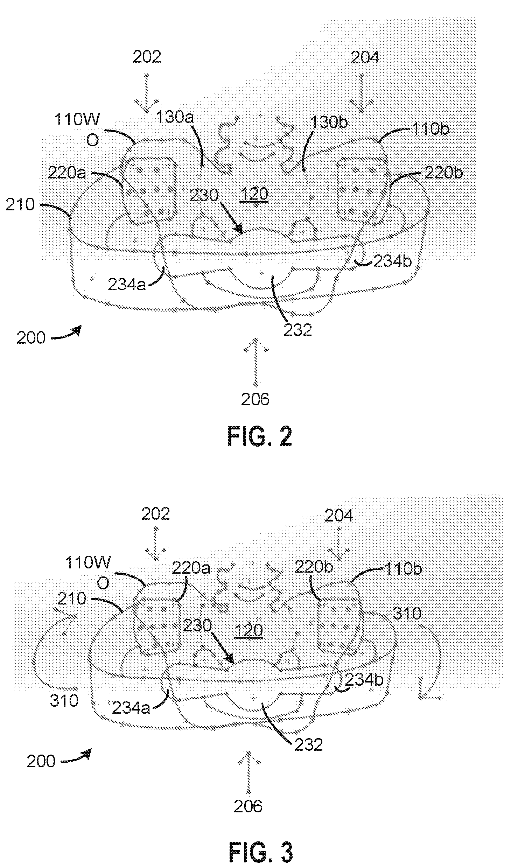

Several example embodiments of a sacroiliac orthosis, brace and/or belt 200 will now be described in more detail in connection with at least FIGs. 2-11, wherein like reference numerals designate the same or corresponding parts throughout the several views. Brace 200 is configured to provide pelvic-sacral support, reduced motion of SI joint(s) 130a, 130b and reduced SI joint pain.

FIGs. 2 and 3 illustrate wireframes of portions of a sacroiliac orthosis, brace and/or belt 200 in an appropriate alignment with portions of the hip region of a user. Brace 200 comprises a belt portion 210 and a plurality of rigid and/or semi-rigid panels 220a, 220b, 230 that are configured to apply pressure in the coronal plane of a user as belt portion 210 is cinched or tightened circumferentially around the user's pelvis and/or low back. In some embodiments, one or more of panels 220a, 220b, 230 may comprise high density polyethylene (HDPE). However, the present disclosure is not so limited and one or more of panels 220a, 220b, 230 may comprise any suitably rigid or semi-rigid material. As illustrated in **FIGs. 2** and **3****,** a first posterior sacral panel 220a is adjustably coupled at a position on belt portion 210 such that first posterior sacral panel 220a is disposed directly posterior of a first posterior side 110a of the ilium of a user when brace 200 is worn. A second posterior sacral panel 220b is adjustably coupled at a different position on belt portion 210 such that second posterior sacral panel 220b is disposed directly posterior of a second posterior side 110b of the ilium of the user when brace 200 is worn. In some embodiments, first and second posterior sacral panels 220a, 220b may have a substantially rectangular form factor, for example, having rounded corners and at least one outwardly bowed or convex side as illustrated. However, the present disclosure is not so limited and first and second sacral panels 220a, 220b may have any suitable form factor.

In some embodiments, brace 200 also comprises a rigid or semirigid, substantially flat anterior abdominal panel 230 coupled to yet another position on belt portion 210 such that anterior abdominal panel 230 is disposed directly against an anterior side of an abdomen of the user when brace 200 is worn.

When brace 200 is properly positioned and belt portion 210 is cinched around the waist, low back and/or hips of the user, brace 200 provides increased pelvic-sacral support. The adjustable pressure system formed by belt portion 210 and at least a subset of panels 220a, 220b, 230 reduces motion about and thereby stabilizes first and/or second SI joints 130a, 130b. For example, as illustrated in **FIGs. 2** and **3****,** when belt portion 210 is tightened along its longitudinal extent (in a circumferential direction about the user's waist, hips and/or low back) a circumferential force 310 is generated about belt portion 210. This causes posterior sacral panels 220a, 220b to be pressed in an anterior direction (denoted by respective arrows 202, 204) and apply a respective force to first and second posterior sides 110a, 110b of ilium of the pelvis in the same anterior direction. Similarly, anterior abdominal panel 230 is pressed in a posterior direction (denoted by arrow 206) and applies a force to the abdomen of the user in the same posterior direction.

As can be seen in **FIGs.** 2 and 3, while each of first and second posterior sacral panels 220a, 220b ultimately provide an anterior force to respective posterior sides of the ilium, to optimize SI joint stability, it may be desirable for abdominal panel 230 to provide this posterior force across a relatively broader area of the abdomen directly anterior of sacrum 120, SI joints 130a, 130b, and at least a portion of first and second sides 110a, 110b of the ilium. Accordingly, in some embodiments, abdominal panel 230 has a substantially "plus", "cross", or "batman emblem"-like shape. For example, in some such embodiments, abdominal panel 230 comprises a central portion 232 and a first and a second extension 234a, 234b, each extending away from central portion 232 in opposite directions. Central portion 232 may have an enlarged, substantially circular or ellipsoid form factor such that central portion 232 bulges with respect to the immediately adjacent portions of first and second extensions 234a, 234b. Such an enlarged form factor allows central portion 232 to distribute a portion of the posterior force across a relatively broader portion of the abdomen directly overlying at least sacrum 120. Similarly, in some embodiments, first and second extensions 234a, 234b may increase in width along their lengths of extension away from central portion 232. Such a form factor allows first and second extensions 234a, 234b to distribute a portion of the posterior force across a relatively broader portion of the abdomen directly anterior of respective SI joints 130a, 130b and at least portions of first and second sides 110a, 110b of ilium along its length of extension. In this way, belt portion 210, posterior sacral panels 220a, 220b and, in some embodiments, anterior abdominal panel 230 work together to apply circumferential forces 310, posterior forces 202, 204 and distributed anterior force 206 to, thereby, stabilize and increase support to SI joints 130a, 130b, specifically.

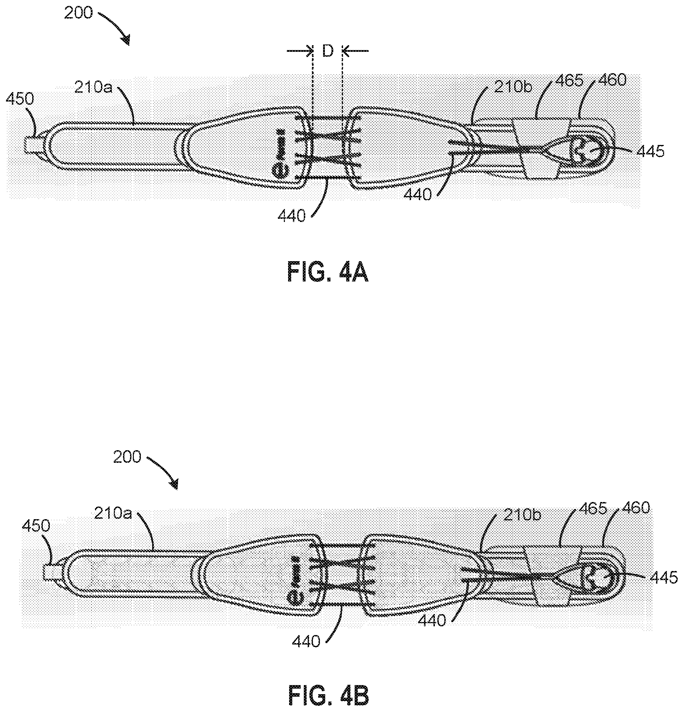

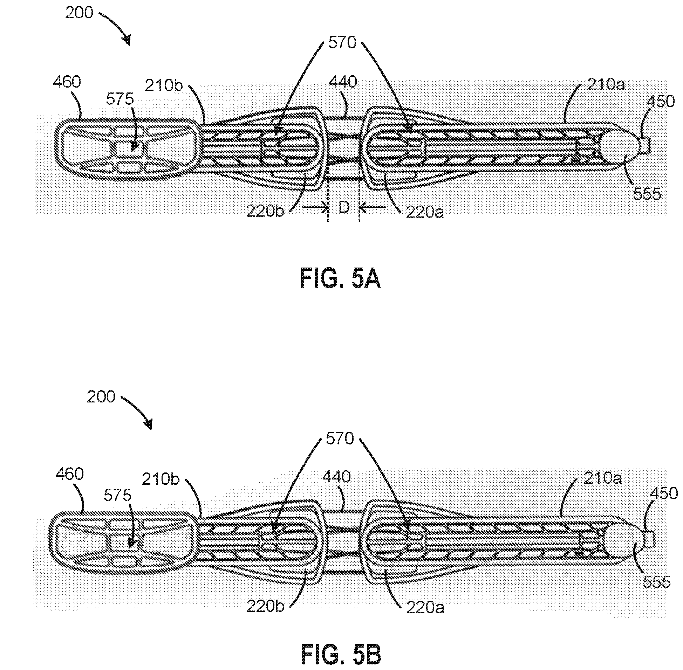

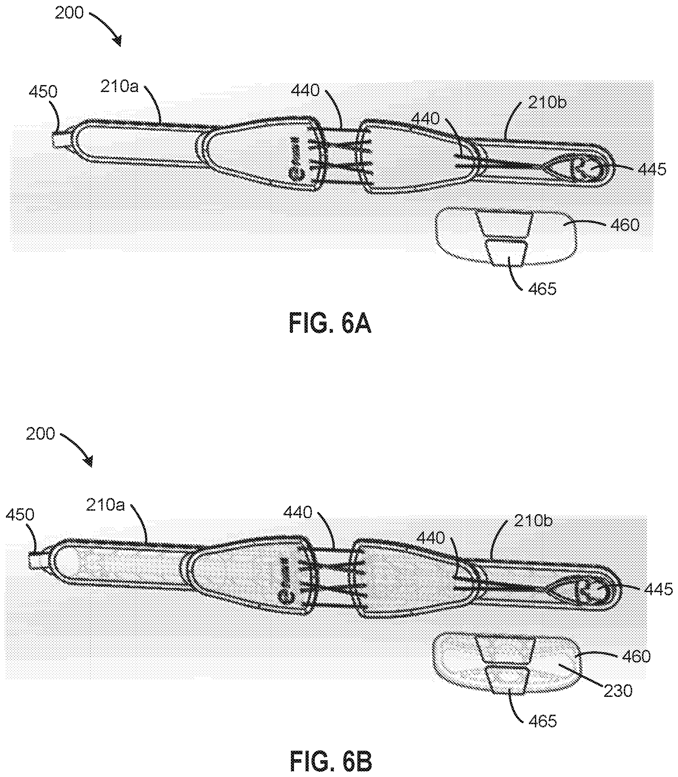

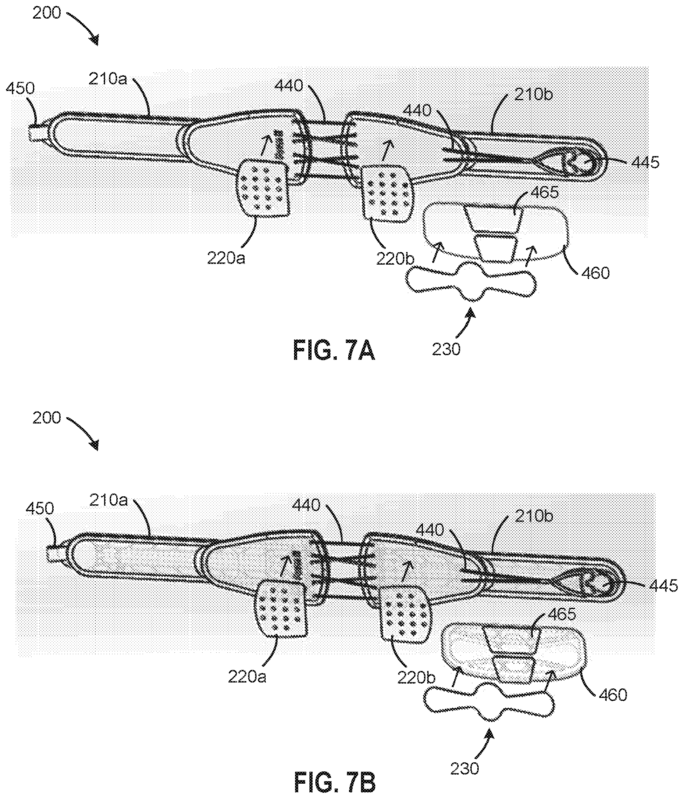

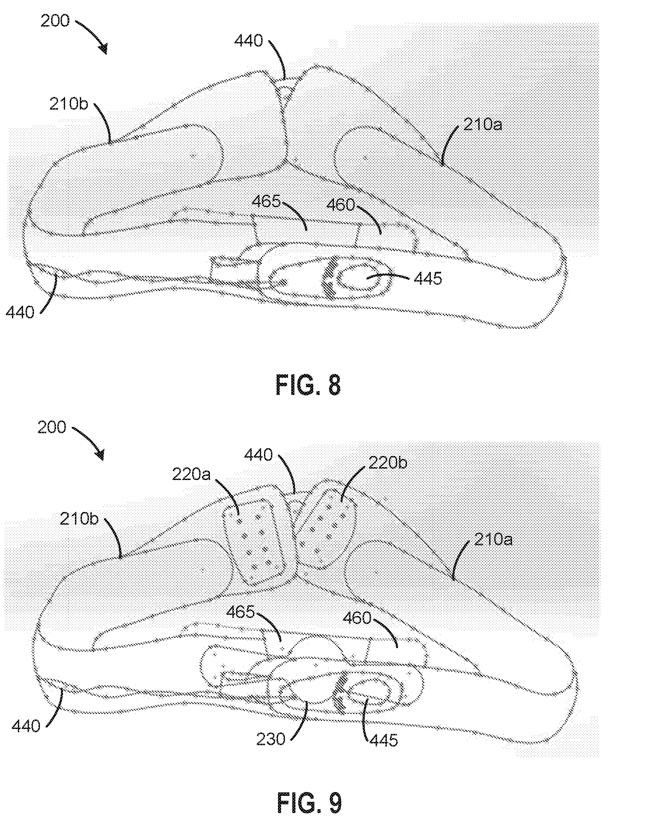

Several detailed aspects of one or more embodiments of brace 200 will now be described in connection with one or more of **FIGs. 4A-11****.** **FIGs. 4A****,** **4B****,** **6A****,** **6B****,** **7A** and **7B** illustrate an outer side of brace 200. **FIGs. 4A** and **4B** illustrate brace 200 with a removable anterior abdominal panel housing 460 attached to belt portion 210. **FIGs. 6A** and **6B** illustrate brace 200 with removable anterior abdominal panel housing 460 detached from belt portion 210. **FIGs. 7A** and **7B** illustrate semi exploded views of brace 200 with removable anterior abdominal panel housing 460 detached from belt portion 210 and panels 220a, 220b, 230 outside of belt portion 210. **FIGs. 5A** and **5B** illustrate an inner side of brace 200. And **FIGs. 8-11** illustrate various views or wireframe illustrations of brace 200 with the second ends of belt portions 220a, 220b attached to one another.

To affect cinching and/or tightening of belt portion 210 along its longitudinal extent, in some embodiments, belt portion 210 can comprise a first belt portion 210a and a second belt portion 210b. In some embodiments, first and second belt portions 210a, 210b are adjustably coupled to one another at respective first ends via a closure system. As illustrated, the closure system may include a plurality of laces 440; however, it will be appreciated by a skilled artisan that any number of conventional orthotic closure systems may be employed. In some of the embodiments where the closure system includes laces 440, laces 440 are configured to provide a predetermined spacing "D" (see **FIGs. 4A** and **5A****)** between the adjacent and facing first ends of first and second belt portions 210a, 210b. In some other embodiments, laces 440 are configured to provide an adjustable spacing "D" between the adjacent and facing first ends of first and second belt portions 210a, 210b (see **FIGs. 4A** and **5A****).**

In some embodiments, laces 440 may be strung back and forth between the adjacent and facing first ends of first and second belt portions 210a, 210b a plurality of times utilizing any suitable features, for example, hooks, loops and/or apertures in one or more portions of first and second belt portions 210a, 210b. For example, the outermost passes of laces 440 may extend between the adjacent and facing first ends of first and second belt portions 210a, 210b along a path that is substantially parallel to the longitudinal extent of belt portions 210a, 210b; while the innermost passes of laces 440 may extend between the adjacent and facing first ends of first and second belt portions 210a, 210b along paths that are askew from the direction substantially parallel to the longitudinal extent of belt portions 210a, 210b such that passes in pairs of the innermost passes of laces 440 cross one another in a substantially X-shaped pattern. Employing both types of run extensions of laces 440 between the adjacent and facing first ends of first and second belt portions 210a, 210b provides both increased cinching ability as well as increased resistance to misalignment between first and second belt portions 210a, 210b when cinched.

In some embodiments, at least a portion of laces 440 may ultimately extend along a portion of second belt portion 210b and attach to an adjustment mechanism 445, for example a pull-tab, configured to adjust the spacing "D" between the adjacent and facing first ends of first and second belt portions 210a, 210b. For example, adjustment mechanism 445 may be configured to decrease the spacing "D" when pull tab 445 is pulled away from the user's body while brace 200 is at least partially secured around the user. In some embodiments, pull tab 445 may be positioned proximate a second end of second belt portion 210b opposite the first end. However, the present disclosure is not so limited and pull tab 445 may be disposed at any suitable location on brace 200. In operation, upon coupling of the second ends of first and second belt portions 210a, 210b to one another around the user's waist, pull tab 445 may be pulled until the desired spacing "D" between the adjacent and facing first ends of first and second belt portions 210a, 210b and circumferential force 310 is achieved. Then pull tab 445 may be secured to second belt portion 210b, for example, via hook and loop fasteners.

Adjusting pull-tab 445 has the effect of potentially providing two simultaneous adjustments to brace 200. First, it adjusts the spacing "D" between the adjacent and facing first ends of first and second belt portions 210a, 210b. Because, as will be described in more detail, first and second posterior sacral panels 220a, 220b are embedded in and/or otherwise secured proximate to the first ends of first and second belt portions 210a, 210b, adjusting the spacing "D" between the adjacent and facing first ends of first and second belt portions 210a, 210b also adjusts a related spacing between first and second posterior sacral panels 220a, 220b. This allows for customization of brace 200 to the specific morphologies of the user. Second, if the second ends of first and second belt portions 210a, 210b are already coupled to one another around a user's waist, adjusting pull-tab 445 will also have the secondary effect of simultaneously adjusting circumferential tension 310 (see FIG. 3) in belt portions 210a, 210b. Since this adjustment of circumferential tension 310 is directly related to the adjustment in the spacing "D", their mutual adjustments are linked.

In some embodiments, first and second belt portions 210a, 210b are additionally or alternatively adjustably coupled to one another at respective second ends via fastening straps 555 (see, **FIGs. 5A, 5B****),** e.g., hook and loop fastening straps. In some embodiments, at least a portion of the second ends of each of first and second belt portions 210a, 210b themselves may comprise such fastening straps and may include complementary hook and loop materials configured to overlap one another and secure first and second belt portions 210a, 210b together. In some embodiments, one or both ends of the fastening straps 555 comprise a gripping feature 450 (e.g., pull tabs, loops or other suitable mechanisms) that allow the user to grasp first and second belt portions 210a, 210b and adjust an amount of circumferential tension 310 by controlling the degree of overlap between the second ends of first and second belt portions 210a, 210b, thereby adjusting an amount of force 202, 204, 206, pressure and stabilizing support provided to SI joints 130a, 130b.

As briefly described above, first and second posterior sacral panels 220a, 220b may be embedded, secured or sewn into a pocket within, and/or otherwise secured proximate to, the first ends of first and second belt portions 210a, 210b (see, e.g., **FIGs. 5A, 5B****,** **7A** and **7B****).** In some embodiments, first and second posterior sacral panels 220a, 220b are removably embedded in and/or otherwise secured to first ends of first and second belt portions 210a, 210b. This removability may aid in cleaning or washing of brace 200. In some other embodiments, first and second posterior sacral panels 220a, 220b are permanently embedded in or sewn into a pocket of, and/or otherwise secured to, first and second belt portions 210a, 210b. Such permanent securement may aid in preventing loss or misplacement of panels 220a, 220b.

In some embodiments, an inner, user-facing surface 570 of belt portions 210a, 210b may be textured and/or patterned to reduce slippage or drifting of belt portions 210a, 210b while brace 200 is worn. For example, as illustrated in **FIGs. 5A** and **5B****,** surface 570 may have a raised or textured pattern that is substantially symmetrical about a midline extending along a length of extension of belt portions 210a, 210b; in some such embodiments and for further example, raised or textured portions of surface 570 symmetrically skewing centrally toward the spacing "D" between belt portions 210a, 210b toward its lateral extents.

In some embodiments, brace 200 comprises anterior abdominal panel housing 460, which is configured to house and/or secure anterior abdominal panel 230 therein (see, e.g., FIG. **6B****).** In some embodiments, abdominal panel housing 460 comprises a thermoformed foam, formed to have a void substantially in the shape of anterior abdominal panel 230. However, the present disclosure is not so limited and abdominal panel housing 460 may comprise any suitable material.

In some embodiments, anterior abdominal panel housing 460 is permanently attached, e.g., sewn, to one of belt portions 210a, 210b. In some other embodiments, anterior abdominal panel housing 460 is removably attached to one of belt portions 210a, 210b, e.g., second belt portion 210b as illustrated in **FIGs. 4A** and **4B****.** Abdominal panel housing 460 may be removably attached to one of belt portions 210a, 210b via a fixing mechanism 465, e.g., hook and loop fasteners, a clip, a strap, a band, a loop, an elastic member, a buckle, or any other suitably adjustable fixing mechanism. In some embodiments, such removable attachment aids in proper placement and adjustment of abdominal panel housing 460 against the abdomen of variously sized and shaped users. For example, in embodiments where fixing mechanism 465 affords such a sliding degree of freedom, abdominal panel housing 460 may be slid along the one of belt portions 210a, 210b to which it is attached until abdominal panel housing 460 is properly positioned. This may be particularly useful where cinching belt portions 210a, 210b around the waist of the user results in misalignment of abdominal panel housing 460 that was initially roughly properly aligned. In embodiments where fixing mechanism 465 does not afford such a sliding degree of freedom (e.g., embodiments where fixing mechanism 465 comprises hook and loop fasteners), abdominal panel housing 460 may be properly fixed to one of belt portions 210a, 210b. In some embodiments, an inner, user-facing side of abdominal panel housing 460 may have a textured and/or patterned surface 575 configured to reduce slippage or drifting of abdominal panel housing 460 while brace 200 is worn.

While embodiments of brace 200 have been described including anterior abdominal panel housing 460 and anterior abdominal panel 230, brace 200 may still be at least partially functional without the use of anterior abdominal panel housing 460 and anterior abdominal panel 230 disposed therein. For example, belt portions 210a, 210b may still be configured to provide sufficient force and pressure against the user's abdomen to suitably stabilize and support SI joints 130a, 130b, as described above, provided they are cinched tightly enough to provide sufficient circumferential force 310 and, so, anterior force 206.

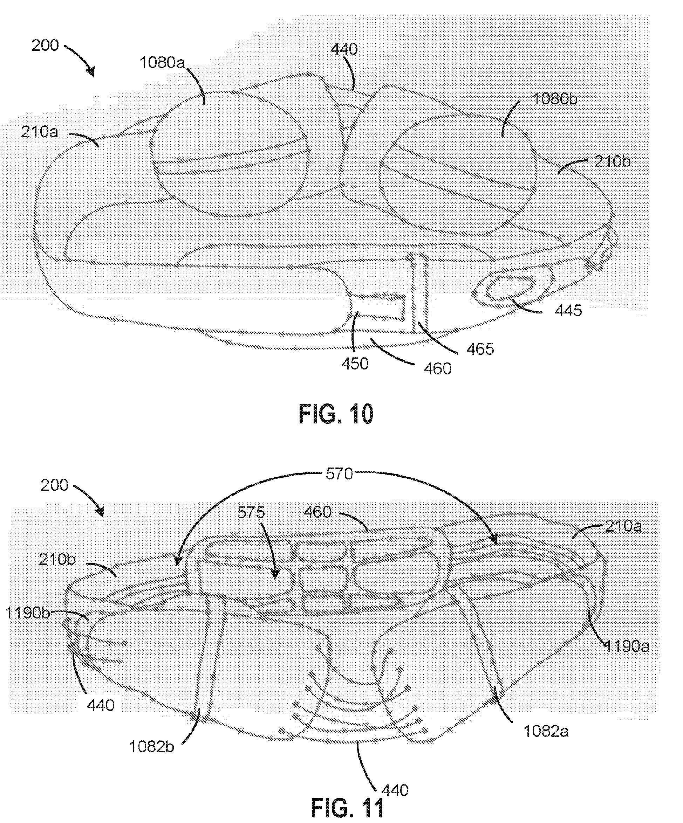

As further illustrated at least in **FIGs. 10** and **11****,** brace 200 may optionally include pads 1080a, 1080b, configured to provide added cushion and a more flexible fit, particularly when disposed on belt portions 210a, 210b between the user's hips and first and second posterior sacral panels 220a, 220b. In some embodiments, pads 1080a, 1080b are configured to be removable, for example, attached utilizing hook and loop fasteners or one or more elastic bands 1082a (see **FIG. 11****)** without taking away the structural support afforded to brace 200 by posterior sacral panels 220a, 220b and, optionally, anterior abdominal panel 230. In some other embodiments, pads 1080a, 1080b are permanently secured, e.g., sewn, to belt portions 210a, 210b.

As further illustrated by at least **FIGs. 10** and **11****,** in some embodiment, first ends of first and second belt portions 210a, 210b may be pivotally coupled to the remainder of their corresponding belt portions 210a, 210b via respective pivoting fixtures 1190a, 1190b. In some such embodiments, pivoting fixtures 1190a, 1190b may be sewn into, or otherwise coupled to, the first ends and remainders of first and second belt portions 210a, 210b. Accordingly, in such embodiments, the user is afforded an additional degree of flexibility in that first ends of first and second belt portions 210a, 210b may extend in slightly different directions than the remainder of their respective belt portions 210a, 210b, as necessitated by the features and contours of the user's unique body - for example in the case where a user has a pendulous abdomen. In some other embodiments, the first ends of first and second belt portions 210a, 210b may be non-pivotally coupled to the remainder of their corresponding belt portions 210a, 210b. In other words, the first ends of first and second belt portions 210a, 210b may be fixedly coupled to, and not be configured to pivot with respect to, the remainder of their corresponding belt portions 210a, 210b beyond that afforded by the intrinsic flexibility of the material(s) or fabric(s) comprising the fixedly coupled first ends and remainders of belt portions 210a, 210b.

In some embodiments, brace 200 can comprise further design features configured to accommodate a pendulous abdomen. In some embodiments, brace 200 can be prefabricated and, thereby, provide an off-the-shelf solution to reduce motion, pain or discomfort about the SI joints 130a, 130b, and/or damage that can result therefrom.

### Example Method(s) of Use

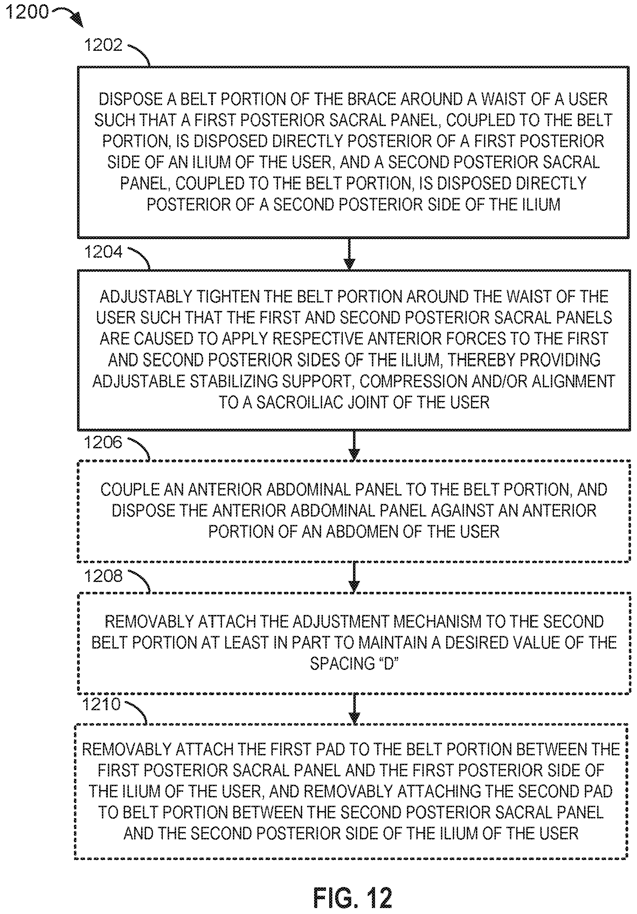

The disclosure now turns to **FIG. 12****,** which illustrates a flowchart 1200 related to an example method of utilizing a sacroiliac orthosis, brace and/or belt to provide pelvic sacral support, as described anywhere in this disclosure.

Although the method(s) disclosed herein comprise(s) one or more steps or actions for achieving the described method(s), such steps and/or actions may be interchanged with one another, and/or a subset of these steps and/or actions may be used, without departing from the scope of the claims. In other words, unless a specific order of steps or actions is specified, the order and/or use of specific steps and/or actions may be modified. One or more additional steps not specifically described herein may also be included.

Step 1202 includes disposing a belt portion of the brace around a waist of a user such that a first posterior sacral panel, coupled to the belt portion, is disposed directly posterior of a first posterior side of an ilium of the user, and a second posterior sacral panel, coupled to the belt portion, is disposed directly posterior of a second posterior side of the ilium.

For example, as previously described in connection with at least one of **FIGs. 2-11****,** belt portion 210 of brace 200 can be disposed around a waist of a user such that first posterior sacral panel 220a, coupled to belt portion 210, is disposed directly posterior of first posterior side 110a of an ilium of the user, and second posterior sacral panel 220b, coupled to belt portion 210, is disposed directly posterior of second posterior side 110b of the ilium.

Step 1204 includes adjustably tightening the belt portion around the waist of the user such that the first and second posterior sacral panels are caused to apply respective anterior forces to the first and second posterior sides of the ilium, thereby providing adjustable stabilizing support, compression and/or alignment to a sacroiliac joint of the user.

For example, as previously described in connection with at least one of **FIGs. 2-11****,** belt portion 210 can be adjustably tightening around the waist of the user such that first and second posterior sacral panels 220a, 220b are caused to apply respective anterior forces 202, 204 to first and second posterior sides 110a, 110b of the ilium, thereby providing adjustable stabilizing support, compression and/or alignment to sacroiliac joint(s) 130a, 130b of the user.

In some embodiments, an optional step 1206 may include coupling an anterior abdominal panel to the belt portion, and disposing the anterior abdominal panel against an anterior portion of an abdomen of the user.

For example, as previously described in connection with at least one of **FIGs. 2-11****,** anterior abdominal panel 230 may be coupled to belt portion 210 and disposed against an anterior portion of an abdomen of the user.

In some embodiments, adjustably tightening belt portion 210 causes anterior abdominal panel 230 to apply posterior force 206 to the abdomen of the user, thereby contributing to the adjustable stabilizing support, compression and/or alignment to sacroiliac joint(s) 130a, 130b of the user.

In some embodiments, coupling anterior abdominal panel 230 to belt portion 210 comprises coupling anterior abdominal housing 460, which holds anterior abdominal panel 230, to belt portion 210, and disposing anterior abdominal panel 230 against the anterior portion of the abdomen of the user comprises disposing anterior abdominal housing 430, which holds anterior abdominal panel 230, to the anterior portion of the abdomen of the user.

In some embodiments, a user-facing surface 575 of the anterior abdominal housing is textured and/or patterned to reduce slippage or drifting of anterior abdominal housing 460 while brace 200 is worn. In some embodiments, anterior abdominal panel 230 is substantially flat and rigid or semi-rigid. In some embodiments, anterior abdominal panel 230 comprises a central portion 232 and first and second extensions 234a, 234b, which each extend away from central portion 32 in opposite directions, and central portion 232 comprises an enlarged, substantially circular or ellipsoid form factor such that central portion 232 bulges with respect to immediately adjacent portions of first and second extensions 234a, 234b. In some embodiments, for each of first and second extensions 234a, 234b, a respective width of the extension increases as the extension extends away from central portion 232. In some embodiments, central portion 232 is configured to be disposed directly anterior of at least sacrum 120 of the user when brace 200 is worn. In some embodiments, each of first and second extensions 234a, 234b are configured to be disposed directly anterior of a respective sacroiliac joint 130a, 130b and at least a portion of the corresponding one of first and second sides 110a, 110b of the ilium of the user when brace 200 is worn.

In some embodiments, belt portion 210 comprises a first belt portion 210a, a second belt portion 210b, and a plurality of laces 440 that couple adjacent and facing first ends of the first and second belt portions with an adjustable spacing (D) therebetween. First posterior sacral panel 220a is coupled proximate to the first end of first belt portion 210a and second posterior sacral panel 220b is coupled proximate to the first end of second belt portion 210b. In some embodiments, first and second posterior sacral panels 220a, 220b are permanently secured within respective pockets disposed proximate to the first end of the corresponding first and second belt portions 210a, 210b. In some embodiments, first and second posterior sacral panels 220a, 220b are removably secured proximate to the first end of the corresponding first and second belt portions 210a, 210b before brace 200 is worn by the user. In some embodiments, first and second posterior sacral panels 220a, 220b have substantially rectangular form factors. In some embodiments, first and second posterior sacral panels 220a, 220b have rounded corners and at least one outwardly bowed or convex side. In some embodiments, first and second posterior sacral panels 220a, 220b are rigid or semi-rigid.

In some embodiments, first and second belt portions 210a, 210b comprise fastening straps 450 at respective second ends and adjustably tightening belt portion 210 around the waist of the user comprises adjusting an amount of tension in belt portion 210 based at least in part on an amount of overlap between the second ends of first and second belt portions 210a, 210b. In some embodiments, fastening straps 450 comprise one or more gripping features 450 and adjustably tightening belt portion 210 around the waist of the user comprises grasping gripping feature 450 while adjusting the amount of overlap between the second ends of first and second belt portions 210a, 210b.

In some embodiments, plurality of laces 440 extend between the adjacent and facing first ends of first and second belt portions 20a, 210b a plurality of times, thereby forming a plurality of passes of the plurality of laces. In some embodiments, a first subset of the plurality of passes of laces 440 extend between the adjacent and facing first ends of first and second belt portions 210a, 210b along a path that is substantially parallel to a longitudinal extent of first and second belt portions 210a, 210b, and a second subset of the plurality of passes of laces 440 extend between the adjacent and facing first ends of first and second belt portions 210a, 210b along paths that are askew from the longitudinal extent of first and second belt portions 20a, 210b such that passes in pairs of the second subset of the plurality of passes cross one another in a substantially X-shaped pattern.

In some embodiments, at least a portion of laces 440 extend along a portion of second belt portion 210b and attach to adjustment mechanism 445. In some embodiments, adjustment mechanism 445 comprises a pull tab and adjustably tightening belt portion 210 around the waist of the user comprises pulling pull tap 445 away from the user's body to decrease the spacing "D" while brace 200 is at least partially secured around the user. In some embodiments, pull tab 445 is configured to simultaneously increase an amount of circumferential tension 310 in first and second belt portions 210a, 210b when pull tab 445 is pulled away from the user's body while brace 200 is at least partially secured around the user. In some embodiments, pull tab 445 is configured to simultaneously decrease a spacing between first and second posterior sacral panels 220a, 220b when pull tab 445 is pulled away from the user's body while brace 200 is at least partially secured around the user.

In some embodiments, an optional step 1208 may include removably attaching the adjustment mechanism to the second belt portion at least in part to maintain a desired value of the spacing "D". For example, as previously described in connection with at least **FIGs. 2-11****,** adjustment mechanism 445 may be removably attached to second belt portion 210b at least in part to maintain a desired value of the spacing "D".

In some embodiments, the first end of each of first and second belt portions 210a, 210b is pivotally coupled to a remainder of the respective first and second belt portion 20a, 210b by a respective pivoting fixture 1190a, 1190b such that the first end of each of first and second belt portions 210a, 210b is configured to extend in slightly different direction than the remainder of the respective belt portion 210a, 210b when brace 200 is worn. In some embodiments, a user-facing surface 570 of first and second belt portions 210a, 210b is textured and/or patterned to reduce slippage or drifting of first and second belt portions 210a, 210b while brace 200 is worn. In some embodiments, the texture and/or pattern of user-facing surface 570 is substantially symmetrical about a midline extending along a length of extension of first and second belt portions 210a, 210b.

In some embodiments, an optional step 1210 may include removably attaching the first pad to the belt portion between the first posterior sacral panel and the first posterior side of the ilium of the user, and removably attaching the second pad to belt portion between the second posterior sacral panel and the second posterior side of the ilium of the user.

For example, as previously described in connection with at least one of **FIGs. 2-11****,** first pad 1080a may be removably attached to belt portion 210a between first posterior sacral panel 220a and first posterior side 110a of the ilium of the user, and second pad 1080b may be removably attached to belt portion 210b between second posterior sacral panel 220b and second posterior side 110b of the ilium of the user.

### Example Methods of Manufacture

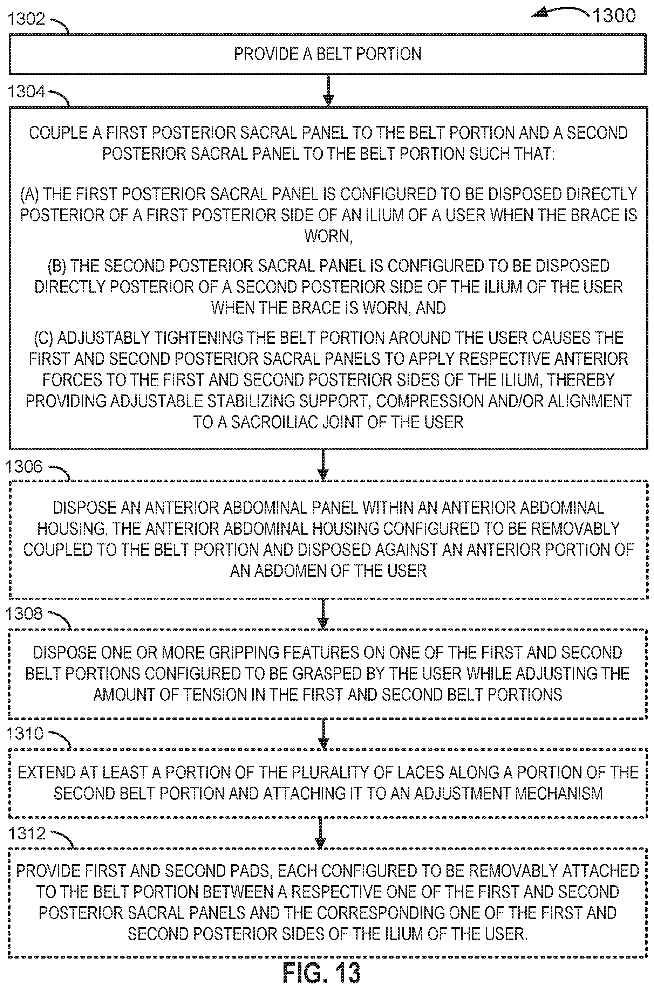

The disclosure now turns to **FIG. 13****,** which illustrates a flowchart 1300 related to an example method of manufacturing a sacroiliac orthosis, brace and/or belt for providing pelvic sacral support, as described anywhere in this disclosure.

Although the method(s) disclosed herein comprise(s) one or more steps or actions for achieving the described method(s), such steps and/or actions may be interchanged with one another, and/or a subset of these steps and/or actions may be used. In other words, unless a specific order of steps or actions is specified, the order and/or use of specific steps and/or actions may be modified. One or more additional steps not specifically described herein may also be included.

Step 1302 includes providing a belt portion. For example, as previously described in connection with at least **FIGs. 2-11****,** belt portion 210 may be provided as described anywhere in this disclosure.

Step 1304 includes coupling a first posterior sacral panel to the belt portion and a second posterior sacral panel to the belt portion such that: (A) the first posterior sacral panel is configured to be disposed directly posterior of a first posterior side of an ilium of a user when the brace is worn, (B) the second posterior sacral panel is configured to be disposed directly posterior of a second posterior side of the ilium of the user when the brace is worn, and (C) adjustably tightening the belt portion around the user causes the first and second posterior sacral panels to apply respective anterior forces to the first and second posterior sides of the ilium, thereby providing adjustable stabilizing support, compression and/or alignment to a sacroiliac joint of the user.

For example, as previously described in connection with at least **FIGs. 2-11****,** first posterior sacral panel 220a may be coupled to belt portion 210a and second posterior sacral panel 220b to belt portion 210b such that: (A) first posterior sacral panel 220a is configured to be disposed directly posterior of first posterior side 110a of an ilium of a user when brace 200 is worn, (B) second posterior sacral panel 220a is configured to be disposed directly posterior of second posterior side 110b of the ilium of the user when brace 200 is worn, and (C) adjustably tightening belt portion 210 around the user causes first and second posterior sacral panels 220a, 220b to apply respective anterior forces 202, 204 to first and second posterior sides 110a, 110b of the ilium, thereby providing adjustable stabilizing support, compression and/or alignment to sacroiliac joint(s) 130a, 130b of the user.

In some embodiments, an optional step 1306 may include disposing an anterior abdominal panel within an anterior abdominal housing, wherein the anterior abdominal housing is configured to be removably coupled to the belt portion and disposed against an anterior portion of an abdomen of the user. For example, as previously described in connection with at least **FIGs. 2-11****,** anterior abdominal panel 230 may be disposed within anterior abdominal housing 460, wherein housing 460 is configured to be removably coupled to belt portion 210 and disposed against an anterior portion of an abdomen of the user.

In some embodiments, anterior abdominal panel 230 is configured to apply a posterior force 206 to the abdomen of the user, thereby contributing to the adjustable stabilizing support, compression and/or alignment to sacroiliac joint(s) 130a, 130b of the user.

In some embodiments, a user-facing surface 575 of anterior abdominal housing 460 may include a texture and/or pattern configured to reduce slippage or drifting of anterior abdominal housing 460 while brace 200 is worn.

In some embodiments, anterior abdominal panel 230 is substantially flat and rigid or semi-rigid. In some embodiments, anterior abdominal panel 230 comprises central portion 232, and first and second extensions 234a, 234b that each extend away from central portion 232 in opposite directions. In some such embodiments, central portion 232 comprises an enlarged, substantially circular or ellipsoid form factor such that central portion 232 bulges with respect to immediately adjacent portions of first and second extensions 234a, 234b. In some embodiments, for each of first and second extensions 234a, 234b, a respective width of the extension increases as the extension extends away from central portion 232. In some embodiments, anterior abdominal panel 230 is disposed within anterior abdominal housing 460 such that central portion 230 of anterior abdominal panel 230 is disposed directly anterior of at least sacrum 120 of the user when brace 200 is worn. In some embodiments, anterior abdominal panel 230 is disposed within anterior abdominal housing 460 such that each of first and second extensions 234a, 234b is disposed directly anterior of a respective sacroiliac joint 130a, 130b and at least a portion of the corresponding one of first and second sides 110a, 110b of the ilium of the user when brace 200 is worn.

In some embodiments, providing belt portion 210 comprises providing first belt portion 210a, providing second belt portion 210b, and coupling adjacent and facing first ends of first and second belt portions 210a, 210b with the adjustable spacing "D" therebetween utilizing laces (440). In some such embodiments coupling first posterior sacral panel 220a to belt portion 210 comprises coupling first posterior sacral panel 220a proximal to the first end of first belt portion 210a, and coupling second posterior sacral panel 220b to belt portion 210 comprises coupling second posterior sacral panel 220b proximal to the first end of second belt portion 210b. In some embodiments, first and second posterior sacral panels 220a, 220b are permanently secured within respective pockets disposed proximate to the first end of the corresponding first and second belt portions 210a, 210b. In some embodiments, first and second posterior sacral panels 220a, 220b are removably secured proximate to the first end of the corresponding first and second belt portions 210a, 210b. In some embodiments, first and second posterior sacral panels 220a, 220b have substantially rectangular form factors. In some embodiments, first and second posterior sacral panels 220a, 220b have rounded corners and at least one outwardly bowed or convex side. In some embodiments, first and second posterior sacral panels 220a, 220b are rigid or semi-rigid.

In some embodiments, first and second belt portions 210a, 210b are each provide with a fastening strap 450 disposed at their respective second ends and configured to adjust an amount of tension in first and second belt portions 210a, 210b based at least in part on an amount of overlap between the second ends of first and second belt portions 20a, 210b.

In some embodiments, an optional step 1308 may include disposing one or more gripping features on one of the first and second belt portions, the one or more gripping features configured to be grasped by the user while adjusting the amount of tension in the first and second belt portions. For example, as previously described in connection with at least **FIGs. 2-11****,** one or more gripping features 450 may be disposed on one of first and second belt portions 210a, 210b and configured to be grasped by the user while adjusting the amount of tension in first and second belt portions 210a, 210b.

In some embodiments, extending laces 440 between the adjacent and facing first ends of first and second belt portions 210a, 210b a plurality of times comprises: (A) extending a first subset of the plurality of passes of laces 440 between the adjacent and facing first ends of first and second belt portions 210a, 210b along a path that is substantially parallel to a longitudinal extent of first and second belt portions 210a, 210b, and (B) extending a second subset of the plurality of passes of laces 440 between the adjacent and facing first ends of first and second belt portions 210a, 210b along paths that are askew from the longitudinal extent of first and second belt portions 210a, 210b such that passes in pairs of the second subset of the plurality of passes cross one another in a substantially X-shaped pattern.

In some embodiments, an optional step 1310 may include extending at least a portion of the plurality of laces along a portion of the second belt portion and attaching the portion of the plurality of laces to an adjustment mechanism. For example, as previously described in connection with at least one of **FIGs. 2-11****,** at least a portion of laces 440 may extend along a portion of second belt portion 210b and attach to adjustment mechanism 445.

In some embodiments, adjustment mechanism 445 comprises a pull tab configured to decrease the spacing "D" when pull tab 445 is pulled away from the user's body while brace 200 is at least partially secured around the user. In some embodiments, pull tab 445 is configured to simultaneously increase an amount of circumferential tension 310 in first and second belt portions 210a, 210b when pull tab 445 is pulled away from the user's body while brace 200 is at least partially secured around the user. In some embodiments, pull tab 445 is configured to simultaneously decrease a spacing between first and second posterior sacral panels 220a, 220b when pull tab 445 is pulled away from the user's body while brace 200 is at least partially secured around the user. In some embodiments, adjustment mechanism 445 is configured to be removably attached to second belt portion 210b at least in part to maintain a desired value of the spacing "D".

In some embodiments, providing first belt portion 210a comprises pivotally coupling the first end of first belt portion 210a to a remainder of first belt portion 210a utilizing a first pivoting fixture 1190a such that the first end of first belt portion 210a is configured to extend in a slightly different direction than the remainder of first belt portion 210a when 200 brace is worn; and providing second belt portion 210b comprises pivotally coupling the first end of second belt portion 210b to a remainder of second belt portion 210b utilizing a second pivoting fixture 1190b such that the first end of second belt portion 210b is configured to extend in a slightly different direction than the remainder of second belt portion 210b when 200 brace is worn.

In some embodiments, user-facing surface 570 of first and second belt portions 210a, 210b is textured and/or patterned to reduce slippage or drifting of first and second belt portions 210a, 210b while brace 200 is worn. In some embodiments, the texture and/or pattern of user-facing surface 570 is substantially symmetrical about a midline extending along a length of extension of first and second belt portions 210a, 210b.

In some embodiments, an optional step 1312 may include providing a first pad and a second pad, each configured to be removably attached to the belt portion between a respective one of the first and second posterior sacral panels and the corresponding one of the first and second posterior sides of the ilium of the user. For example, as previously described in connection with at least one of **FIGs. 2-11****,** first and second pads 1080a, 1080b may be provided such that each is configured to be removably attached to belt portion 210 between a respective one of first and second posterior sacral panels 220a, 220b and the corresponding one of first and second posterior sides 110a, 110b of the ilium of the user.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the scope of the present disclosure as defined by the appended claims.

## Claims

1. An orthopedic sacroiliac brace (200) comprising:
a belt portion (210);
a first posterior sacral panel (220a) coupled to the belt portion and configured to be disposed directly posterior of a first posterior side (110a) of an ilium of a user;
a second posterior sacral panel (220b) coupled to the belt portion and configured to be disposed directly posterior of a second posterior side (110b) of the ilium, and
an anterior abdominal panel (230) coupled to the belt portion and configured to be disposed against an anterior portion of an abdomen of the user, the anterior abdominal panel **characterised by**:
a central portion (232) having a substantially circular or ellipsoid form factor and configured to be disposed directly anterior of at least the sacrum of the user; and
first and second extensions (234a, 234b) that each extend away from the central portion in opposite directions and are configured to be disposed directly anterior of a respective sacroiliac joint and at least a portion of the corresponding side of the ilium;
wherein adjustably tightening the belt portion around the user causes the first and second posterior sacral panels to apply respective anterior forces (202, 204) to the first and second posterior sides of the ilium, and the anterior abdominal panel to simultaneously apply a posterior force (206) to the abdomen of the user, thereby providing adjustable stabilizing support, compression and/or alignment to a sacroiliac joint (130a, 130b) of the user.

2. The brace of any one of claim 1, wherein the anterior abdominal panel is disposed within an anterior abdominal housing configured to be removably or adjustably coupled to the belt portion.

3. The brace of claim 2, wherein a user-facing surface of the anterior abdominal housing is textured and/or patterned to reduce slippage or drifting of the anterior abdominal housing while the brace is worn.

4. The brace of claim 1, wherein, for each of the first and second extensions, a respective width of the extension increases as the extension extends away from the central portion.

5. The brace of any one of claims 1-4, wherein the belt portion comprises:
a first belt portion (210a);
a second belt portion (210b); and
a plurality of laces (440) that couple adjacent and facing first ends of the first and second belt portions with an adjustable spacing (D) therebetween,
the first posterior sacral panel being coupled proximate to the first end of the first belt portion and the second posterior sacral panel being coupled proximate to the first end of the second belt portion.

6. The brace of claim 5, wherein the first and second posterior sacral panels are permanently secured within respective pockets disposed proximate to the first end of the corresponding first and second belt portions.

7. The brace of any one of claims 5 or 6, wherein the first and second posterior sacral panels are removably secured proximate to the first end of the corresponding first and second belt portions.

8. The brace of any one of claims 1-7, wherein the first and second posterior sacral panels comprise at least one of:
substantially rectangular form factors, and
rounded corners and at least one outwardly bowed or convex side.

9. The brace of any one of claims 5-8, wherein the first and second belt portions comprise fastening straps (450) at respective second ends configured to adjust an amount of tension in the belt portion based at least in part on an amount of overlap between the second ends of the first and second belt portions and, thereby, contribute to the adjustable stabilizing support, compression and/or alignment to the sacroiliac joint, the fastening straps comprise one or more gripping features (450) configured to be grasped by the user while adjusting the amount of tension in the first and second belt portions.

10. The brace of any one of claims 5-9, wherein the plurality of laces extend between the adjacent and facing first ends of the first and second belt portions a plurality of times, thereby forming a plurality of passes of the plurality of laces.

11. The brace of claim 10, wherein:
a first subset of the plurality of passes of the plurality of laces extend between the adjacent and facing first ends of the first and second belt portions along a path that is substantially parallel to a longitudinal extent of the first and second belt portions; and
a second subset of the plurality of passes of the plurality of laces extend between the adjacent and facing first ends of the first and second belt portions along paths that are askew from the longitudinal extent of the first and second belt portions such that passes in pairs of the second subset of the plurality of passes cross one another in a substantially X-shaped pattern.

12. The brace of any one of claims 10 or 11, wherein at least a portion of the plurality of laces extend along a portion of the second belt portion and attach to an adjustment mechanism (445).

13. The brace of claim 12, wherein the adjustment mechanism comprises a pull tab configured to decrease the spacing "D" when the pull tab is pulled away from the user's body while the brace is at least partially secured around the user.

14. The brace of claim 13, wherein, while the brace is at least partially secured around the user, the pull tab is configured to simultaneously:
increase an amount of circumferential tension in the first and second belt portions when the pull tab is pulled away from the user's body;[[.]] and
decrease a spacing between the first and second posterior sacral panels when the pull tab is pulled away from the user's body.

15. The brace of any one of claims 5-14, wherein a user-facing surface (570) of the first and second belt portions is textured and/or patterned substantially symmetrically about a midline extending along a length of extension of the first and second belt portions to reduce slippage or drifting of the first and second belt portions while the brace is worn.

## Patentansprüche

1. Orthopädische Iliosakralstütze (200) mit:
einem Gurtteil (21);
einer ersten hinteren Kreuzbeinplatte (220a), die mit dem Gurtteil gekoppelt ist und dazu ausgebildet ist, direkt hinter einer ersten Hinterseite (110a) eines Darmbeins eines Benutzers angeordnet zu werden;
einer zweiten hinteren Kreuzbeinplatte (220b), die mit dem Gurtteil gekoppelt ist und dazu ausgebildet ist, direkt hinter einer zweiten Hinterseite (110b) des Darmbeins angeordnet zu werden, und
einer vorderen Unterleibsplatte (230), die mit dem Gurtteil gekoppelt ist und dazu ausgebildet ist, gegen einen vorderen Teil eines Unterleibs des Benutzers angeordnet zu werden, wobei die vordere Unterleibsplatte **gekennzeichnet ist durch**:
einen zentralen Teil (232) mit einem im Wesentlichen kreisförmigen oder ellipsoiden Formfaktor und ausgebildet, direkt vor mindestens dem Kreuzbein des Benutzers angeordnet zu werden, und
eine erste und eine zweite Verlängerung (234a, 234b), die sich jeweils weg von dem zentralen Teil in entgegengesetzte Richtungen erstrecken und dazu ausgebildet sind, direkt vor einem jeweiligen Iliosakralgelenk und mindestens einem Teil der entsprechenden Seite des Darmbeins angeordnet zu werden;
bei der einstellbar Anziehen des Gurtteils um den Benutzer bewirkt, dass die erste und die zweite hintere Kreuzbeinplatte jeweilige vordere Kräfte (202, 204) auf die erste und die zweite Hinterseite des Darmbeins aufbringen und die vordere Unterleibsplatte gleichzeitig eine hintere Kraft (206) auf den Unterleib des Benutzers aufbringt, wodurch eine einstellbare stabilisierende Unterstützung, Kompression und/oder Ausrichtung für ein Iliosakralgelenk (130a, 130b) des Benutzers bereitgestellt wird.

2. Stütze nach einem von Anspruch 1, bei dem die vordere Unterleibsplatte in einem vorderen Unterleibsgehäuse angeordnet ist, das dazu ausgebildet ist, abnehmbar oder einstellbar mit dem Gurtteil gekoppelt zu werden.

3. Stütze nach Anspruch 2, bei der eine zu einem Benutzer zeigende Oberfläche des vorderen Unterleibsgehäuses zum Verringern eines Rutschens oder Verschiebens des vorderen Unterleibsgehäuses, während die Stütze getragen wird, texturiert und/oder strukturiert ist.

4. Stütze nach Anspruch 1, bei der, für jede von der ersten und der zweiten Verlängerung, eine jeweilige Breite der Verlängerung zunimmt, wenn sich die Verlängerung von dem zentralen Teil weg erstreckt.

5. Stütze nach einem der Ansprüche 1-4, bei der der Gurtteil aufweist:
einen ersten Gurtteil (210a);
einen zweiten Gurtteil (210b); und
mehrere Schnüre (440), die benachbarte und gegenüberliegende erste Enden des ersten und des zweiten Gurtteils mit einem einstellbaren Abstand (D) zwischen diesen koppeln,
wobei die erste hintere Kreuzbeinplatte in der Nähe des ersten Endes des ersten Gurtteils gekoppelt ist und die zweite hintere Kreuzbeinplatte in der Nähe des ersten Endes des zweiten Gurtteils gekoppelt ist.

6. Stütze nach Anspruch 5, bei der die erste und die zweite hintere Kreuzbeinplatte permanent in jeweiligen Taschen, die in der Nähe des ersten Endes der entsprechenden ersten und zweiten Gurtteile angeordnet sind, befestigt sind.

7. Stütze nach einem der Ansprüche 5 oder 6, bei der die erste und die zweite hintere Kreuzbeinplatte in der Nähe des ersten Endes der entsprechenden ersten und zweiten Gurtteile abnehmbar befestigt sind.

8. Stütze nach einem der Ansprüche 1-7, bei der die erste und die zweite hintere Kreuzbeinplatte mindestens eines der Folgenden aufweisen:
im Wesentlichen rechtwinklige Formfaktoren und
abgerundete Ecken und mindestens eine nach außen gebeugte oder konvexe Seite.

9. Stütze nach einem der Ansprüche 5-8, bei der der erste und der zweite Gurtteil Befestigungsriemen (450) an jeweiligen zweiten Enden aufweisen, die zum Einstellen eines Ausmaßes einer Spannung des Gurtteils basierend zumindest zum Teil auf einem Ausmaß eines Überlapps zwischen den zweiten Enden des ersten und zweiten Gurtteils und dadurch Beitragen zu der einstellbaren stabilisierenden Unterstützung, Kompression und/oder Ausrichtung des Iliosakralgelenks ausgebildet sind, wobei die Befestigungsriemen ein oder mehrere Greifmerkmale (450) aufweisen, die dazu ausgebildet sind, von dem Benutzer gegriffen zu werden, während das Ausmaß einer Spannung des ersten und des zweiten Gurtteils eingestellt wird.

10. Stütze nach einem der Ansprüche 5-9, bei der sich die mehreren Schnüre mehrmals zwischen den benachbarten und gegenüberliegenden ersten Enden des ersten und des zweiten Gurtteils erstrecken, wodurch mehrere Durchgänge der mehreren Schnüre ausgebildet werden.

11. Stütze nach Anspruch 10, bei der:
sich eine erste Untergruppe der mehreren Durchgänge der mehreren Schnüre zwischen den benachbarten und gegenüberliegenden ersten Enden des ersten und des zweiten Gurtteils entlang eines Pfads erstrecken, der im Wesentlichen parallel zu einer Längserstreckung des ersten und des zweiten Gurtteils ist; und
sich eine zweite Untergruppe der mehreren Durchgänge der mehreren Schnüre zwischen den benachbarten und gegenüberliegenden ersten Enden des ersten und des zweiten Gurtteils entlang Pfaden erstrecken, die bezüglich der Längserstreckung des ersten und des zweiten Gurtteils schräg verlaufen, so dass Durchgänge von Paaren der zweiten Untergruppe der mehreren Durchgänge einander in einem im Wesentlichen X-förmigen Muster kreuzen.

12. Stütze nach einem der Ansprüche 10 oder 11, bei der sich mindestens ein Teil der mehreren Schnüre entlang eines Teils des zweiten Gurtteils erstreckt und an einem Einstellmechanismus (445) befestigt ist.

13. Stütze nach Anspruch 12, bei der der Einstellmechanismus eine Zuglasche aufweist, die zum Verringern des Abstands "D", wenn die Zuglasche weg von dem Körper des Benutzers gezogen wird, während die Stütze zumindest teilweise um den Benutzer befestigt ist, ausgebildet ist.

14. Stütze nach Anspruch 13, bei der, während die Stütze zumindest teilweise um den Benutzer befestigt ist, die Zuglasche dazu ausgebildet ist, gleichzeitig:
ein Ausmaß einer Umfangsspannung des ersten und des zweiten Gurtteils zu erhöhen, wenn die Zuglasche weg von dem Körper des Benutzers gezogen wird; und
einen Abstand zwischen der ersten und der zweiten hinteren Kreuzbeinplatte zu verringern, wenn die Zuglasche weg von dem Körper des Benutzers gezogen wird.

15. Stütze nach einem der Ansprüche 5-14, bei der eine zu einem Benutzer zeigende Oberfläche (570) des ersten und zweiten Gurtteils im Wesentlichen symmetrisch um eine Mittellinie, die sich entlang einer Länge einer Erstreckung des ersten und des zweiten Gurtteils erstreckt, texturiert und/oder strukturiert ist, so dass ein Rutschen oder Verschieben des ersten und zweiten Gurtteils, während die Stütze getragen wird, verringert wird.

## Revendications

1. Appareil orthopédique (200) sacro-iliaque comprenant :
une partie de ceinture (210) ;
un premier panneau sacré postérieur (220a) couplé à la partie de ceinture et configuré pour être disposé directement à l'arrière d'un premier côté postérieur (110a) d'un ilion d'un utilisateur ;
un second panneau sacré postérieur (220b) couplé à la partie de ceinture et configuré pour être disposé directement à l'arrière d'un second côté postérieur (110b) de l'ilion, et
un panneau abdominal antérieur (230) couplé à la partie de ceinture et configuré pour être disposé contre une partie antérieure de l'abdomen de l'utilisateur, le panneau abdominal antérieur étant **caractérisé par** :
une partie centrale (232) présentant un facteur de forme sensiblement circulaire ou ellipsoïdal et configurée pour être disposée directement en avant d'au moins le sacrum de l'utilisateur ; et
des première et seconde extensions (234a, 234b) qui s'étendent chacune à l'opposé de la partie centrale dans des directions opposées et sont configurées pour être disposées directement en avant d'une articulation sacro-iliaque respective et d'au moins une partie du côté correspondant de l'ilion ;
dans lequel le serrage réglable de la partie de ceinture autour de l'utilisateur amène les premier et second panneaux sacrés postérieurs à exercer des forces antérieures (202, 204) respectives sur les premier et second côtés postérieurs de l'ilion, et le panneau abdominal antérieur à exercer simultanément une force postérieure (206) sur l'abdomen de l'utilisateur, fournissant ainsi un soutien stabilisateur, une compression et/ou un alignement réglables à une articulation sacro-iliaque (130a, 130b) de l'utilisateur.

2. Appareil orthopédique selon l'une quelconque de la revendication 1, dans lequel le panneau abdominal antérieur est disposé à l'intérieur d'un boîtier abdominal antérieur configuré pour être couplé de manière amovible ou réglable à la partie de ceinture.

3. Appareil orthopédique selon la revendication 2, dans lequel une surface tournée vers l'utilisateur du boîtier abdominal antérieur est texturée et/ou à motifs afin de réduire le glissement ou la déviation du boîtier abdominal antérieur lorsque l'appareil orthopédique est porté.

4. Appareil orthopédique selon la revendication 1, dans lequel, pour chacune des première et seconde extensions, une largeur respective de l'extension augmente à mesure que l'extension s'éloigne de la partie centrale.

5. Appareil orthopédique selon l'une quelconque des revendications 1 à 4, dans lequel la partie de ceinture comprend :
une première partie de ceinture (210a) ;
une seconde partie de ceinture (210b) ; et
une pluralité de lacets (440) qui relient les premières extrémités adjacentes et opposées des première et secondes parties de ceinture avec un espacement réglable (D) entre elles,
le premier panneau sacré postérieur étant relié à proximité de la première extrémité de la première partie de ceinture et le second panneau sacré postérieur étant relié à proximité de la première extrémité de la seconde partie de ceinture.

6. Appareil orthopédique selon la revendication 5, dans lequel les premier et second panneaux sacrés postérieurs sont fixés de manière permanente dans des poches respectives disposées à proximité de la première extrémité des première et seconde parties de ceinture correspondantes.

7. Appareil orthopédique selon l'une quelconque des revendications 5 ou 6, dans lequel les premier et second panneaux sacrés postérieurs sont fixés de manière amovible à proximité de la première extrémité des première et seconde parties de ceinture correspondantes.

8. Appareil orthopédique selon l'une quelconque des revendications 1 à 7, dans lequel les premier et second panneaux sacrés postérieurs comprennent au moins l'un parmi :
des facteurs de forme sensiblement rectangulaires, et
des coins arrondis et au moins un côté bombé vers l'extérieur ou convexe.

9. Appareil orthopédique selon l'une quelconque des revendications 5 à 8, dans lequel les première et seconde parties de ceinture comprennent des bandes de fixation (450) au niveau des secondes extrémités respectives, configurées pour régler la tension dans la partie de ceinture en fonction, au moins en partie, du degré de chevauchement entre les secondes extrémités des première et seconde parties de ceinture et, ainsi, contribuer au soutien stabilisateur, à la compression et/ou à l'alignement réglables de l'articulation sacro-iliaque, les bandes de fixation comprennent une ou plusieurs caractéristiques de préhension (450) configurées pour être saisies par l'utilisateur lors du réglage de la tension dans les première et seconde parties de ceinture.

10. Appareil orthopédique selon l'une quelconque des revendications 5 à 9, dans lequel la pluralité de lacets s'étend entre les premières extrémités adjacentes et opposées des première et seconde parties de ceinture à plusieurs reprises, formant ainsi une pluralité de passages de la pluralité de lacets.

11. Appareil orthopédique selon la revendication 10, dans lequel :
un premier sous-ensemble de la pluralité de passages de la pluralité de lacets s'étend entre les premières extrémités adjacentes et opposées des première et seconde parties de ceinture le long d'une trajectoire qui est sensiblement parallèle à une étendue longitudinale des première et seconde parties de ceinture ; et
un second sous-ensemble de la pluralité de passages de la pluralité de lacets s'étend entre les premières extrémités adjacentes et opposées des première et seconde parties de ceinture le long de trajectoires qui sont obliques par rapport à l'étendue longitudinale des première et seconde parties de ceinture, de sorte que les passages par paires du second sous-ensemble de la pluralité de passages se croisent les uns les autres selon un motif sensiblement en forme de X.

12. Appareil orthopédique selon l'une quelconque des revendications 10 ou 11, dans lequel au moins une partie de la pluralité de lacets s'étend le long d'une partie de la seconde partie de ceinture et se fixe à un mécanisme de réglage (445).

13. Appareil orthopédique selon la revendication 12, dans lequel le mécanisme de réglage comprend une languette de traction configurée pour réduire l'espacement « D » lorsque la languette de traction est tirée à l'écart du corps de l'utilisateur alors que l'appareil orthopédique est au moins partiellement fixé autour de l'utilisateur.

14. Appareil orthopédique selon la revendication 13, dans lequel, lorsque l'appareil orthopédique est au moins partiellement fixé autour de l'utilisateur, la languette de traction est configurée pour simultanément :
augmenter la tension circonférentielle dans les première et seconde parties de ceinture lorsque la languette de traction est tirée à l'écart du corps de l'utilisateur ;[[.]] et
diminuer l'espacement entre les premier et second panneaux sacrés postérieurs lorsque la languette de traction est tirée à l'écart du corps de l'utilisateur.

15. Appareil orthopédique selon l'une quelconque des revendications 5 à 14, dans lequel une surface (570) tournée vers l'utilisateur des première et seconde parties de ceinture est texturée et/ou à motifs de manière sensiblement symétrique par rapport à une ligne médiane s'étendant le long d'une longueur de l'étendue des première et seconde parties de ceinture afin de réduire le glissement ou la déviation des première et seconde parties de ceinture lorsque l'appareil orthopédique est porté.
